# EUROPEAN PATENT SPECIFICATION

(11) **EP 2 438 920 B1**
(45) Date of publication and mention of the grant of the patent: **28.09.2016**
(21) Application number: 11184366.0
(22) Date of filing: 07.10.2011
(51) Int. Cl.: A61K 31/554

(54) **CONTROLLED-RELEASE FORMULATIONS OF QUETIAPINE**
QUETIAPIN-ZUSAMMENSETZUNGEN MIT KONTROLLIERTER FREISETZUNG
COMPOSITION PHARMACEUTIQUE À LIBÉRATION CONTRÔLÉE DE QUETIAPINE

(30) Priority: 08.10.2010 TR 201008261
(43) Date of publication of application: 11.04.2012
(60) Divisional application: 16170271.7
(73) Proprietor: SANOVEL ILAC SANAYI VE TICARET A.S., 34460 Sariyer/Istanbul (TR)
(72) Inventor: Cifter, Ümit, 34398 Istanbul (TR); Türkyilmaz, Ali, 34398 Istanbul (TR); Yildirim, Ediz, 34398 Istanbul (TR); Erdem, Yelda, 34398 Istanbul (TR); Ergenekon, Ercan, 34398 Istanbul (TR); Öner, Levent, 34398 Istanbul (TR)
(74) Representative: Vossius & Partner Patentanwälte Rechtsanwälte mbB

(56) References cited:
- EP-A2- 2 153 834
- WO-A2-2010/089259
- US-A1- 2007 244 093
- US-A1- 2009 220 593
- US-A1- 2009 264 408
- "AQUALON Ethylcellulose - Product Data", INTERNET CITATION, 2008, XP002620408, Retrieved from the Internet: URL:http://www.signetchem.com/downloads/da tasheets/Ashland-Aqualon/PDS-Aqualon-EC-sp ecifications.pdf [retrieved on 2011-02-03]
- ROWE ET AL: "The effect of the molecular weight of ethyl cellulose on the drug release properties of mixed films of ethyl cellulose and hydroxypropylmethylcellulose", INTERNATIONAL JOURNAL OF PHARMACEUTICS, ELSEVIER BV, NL, vol. 29, no. 1, 1 March 1986 (1986-03-01), pages 37-41, XP025502228, ISSN: 0378-5173, DOI: DOI:10.1016/0378-5173(86)90197-3 [retrieved on 1986-03-01]
- P.K. KATIKANEMI ET AL.: "Ethylcellulose matrix controlled release tabletds of a water-soluble drug", INTERNATIONAL JOURNAL OF PHARMACEUTICS, ELSEVIER BV, NL, vol. 123, 1995, pages 119-125, XP002620409,

## Description

### Field of Invention

The present invention relates to a novel pharmaceutical formulation comprising quetiapine or a pharmaceutically acceptable salt, solvate or hydrate thereof. The invention more particularly relates to a controlled-release formulation of quetiapine, uniformly dispersed in a matrix tablet and used as a single daily dose.

### Background of Invention

Quetiapine fumarate, a dibenzothiazepine derivative, is an atypical antipsychotic. It ameliorates the negative and positive symptoms of schizophrenia, without giving rise to extrapiramidal side effects. Similar to clozapine, quetiapine shows moderate dopamine D2-receptor antagonist and potent 5-HT2-receptor antagonist effects. The chemical designation of quetiapine fumarate is 2-[2-(4-dibenzo[b,f][1,4]thiazepine-11-yl-1-piperazinyl)ethoxy] ethanol fumarate, with the following chemical structure of Formula I.

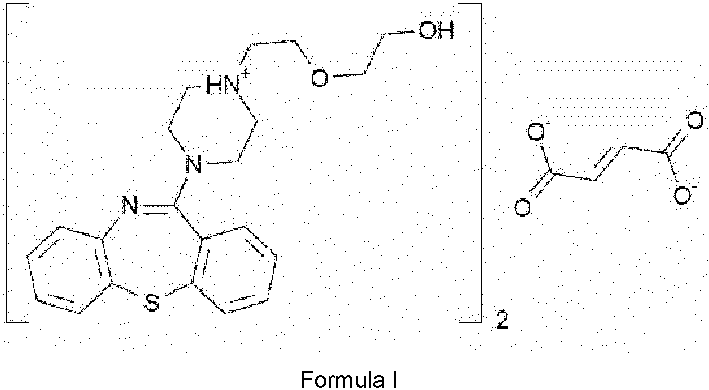

Extended release tablets of quetiapine is marketed under the name Seroquel XR^{®} and is administered orally once in a day. These tablets comprise 50 mg, 150 mg, 200 mg, 300 mg or 400 mg quetiapine fumarate as an active agent.

The quetiapine molecule was disclosed in the applications EP0240228 and US4879288. EP282236 discloses a process for the preparation of a quetiapine molecule.

The patent EP2153834 discloses an extended release multiparticulate formulation comprising quetiapine or a pharmaceutical acceptable salt thereof and an excipient and hydrophobic release controlling agent.

The patent US20080287418 discloses an extended release formulation comprising 9.6 - 10.4% quetiapine, 30% hydroxypropyl methyl cellulose, and 7.2% sodium citrate dihydrate.

The patent WO2010012490 discloses a controlled release formulation comprising quetiapine and a non-gelling carrageenan.

The patent WO9745124 discloses a formulation comprising gelling agent, quetiapine, and one or more excipients. There are mentioned on various polymers in that patent for use as a gelling agent. Hydroxypropyl methyl cellulose, hydroxypropyl cellulose, hydroxymethyl cellulose, hydroxyethyl cellulose, carbomer, sodium carboxymethyl cellulose, polyvinylpyrrolidone, ethyl cellulose, methyl cellulose, carboxymethyl cellulose are specified in that document as gelling agents.

It is a desired feature to obtain an active pharmaceutical agent in a modified-release form in the treatment of many diseases. What is desired with a modified release is to reach desired plasma levels of an active agent of interest throughout a determined period of time, and to provide the drug release at a uniform and constant rate. Thus, the drug administration frequency can be lowered.

There are various difficulties associated with developing a controlled-release formulation. One of the problems encountered is dose damping, by which a drug obtained is released too rapidly. Obtaining intended solubility profiles is also difficult, i.e. it is difficult to control the release rate. For this reason, there may occur variations in the plasma concentrations of active agents, which may lead to toxicity. In addition, there are also daily alterations possible for the active agent in plasma.

There are various formulations available, which have been developed with quetiapine fumarate. There are various problems, however, associated with quetiapine fumarate formulations.

Quetiapine fumarate has a low dissolution rate. Producing controlled-release tablets of quetiapine fumarate and similar active agents having low solubility rates, and providing desired release profiles in these tablets are quite difficult. Generating release amounts at desired intervals bears vital importance with respect to patients. Obtaining a desired release profile depends on the convenience of the excipients to be selected.

Quetiapine fumarate is also a low-density substance. Therefore the tablet production process of quetiapine fumarate is quite difficult. Tablets produced are prone to erosion and disintegration.

Quetiapine fumarate is to be stored below temperatures of 30°C under normal conditions. Stability-related problems are encountered in temperatures exceeding this point. Failing to prepare a formulation with convenient excipients leads to stability problems.

Accordingly, there is a need towards modified-release formulations of quetiapine or a pharmaceutically-acceptable salt thereof, which would overcome the problems referred to hereinabove, and would provide drug plasma concentrations equivalent or better than those of the currently-used immediate-release tablet formulations. The formulation according to the present invention provides a novel modified-release formulation, not disclosed in the relevant art yet.

The modified-release formulation according to the present invention both provides plasma levels comparable to the immediate-release form thereof, and gives an advantageous feature of administering the active drug less frequently, e.g. once or twice a day.

Due to the reasons specified above, there is a need towards a stable pharmaceutical formulation of quetiapine or pharmaceutically acceptable salts, solvates, or hydrates thereof, providing a proper content uniformity and a desired release profile.

### Object and Brief Description of Invention

The present invention relates to a controlled-release formulation of quetiapine, eliminating all aforesaid problems and brining additional advantages to the relevant prior art.

Accordingly, the main object of the present invention is to obtain a controlled-release formulation comprising quetiapine, which is stable and provides a desired release profile.

Another object of the present invention is to obtain a controlled-release formulation with a desired dissolution rate thanks to employing proper excipients.

A further object of the present invention is to ensure a uniform distribution of active agent and excipients in the formulation, so that a tablet form thereof will not be prone to erosion.

A controlled-release pharmaceutical formulation comprising quetiapine or a pharmaceutically acceptable salt, solvate, or polymorph thereof has been developed to carry out all objects, referred to above and to emerge from the following detailed description.

According to a preferred embodiment of the present invention, said novelty is carried out with non-gelling ethyl cellulose with a ethoxyl content in between 48.0 to 52.5%. Thanks to the present invention developed, a stable controlled-release formulation is surprisingly obtained thanks to making use of ethyl cellulose with an ethoxyl content of 48 - 52.5% in which quetiapine is uniformly distributed, thereby solving said problems and allowing to achieve the desired release profile. Thanks to the formulation developed, the risk of dose damping is eliminated and a controlled-release formulation is obtained by using non-gelling ethyl cellulose.

According to a preferred embodiment of the present invention, as much as 35%, preferably as much as 30% of quetiapine or a pharmaceutically acceptable salt thereof is released in 2 hours; 30 to 65%, preferably 35 to 55% of quetiapine or a pharmaceutically acceptable salt thereof is released in 4 hours; and a minimum of 80% of quetiapine or a pharmaceutically acceptable salt thereof is released in 16 hours.

According to a preferred embodiment of the present invention, the weight ratio of quetiapine to ethyl cellulose is between 0.1 to 10, preferably 1 to 4, and more preferably 1.5 to 3.

According to a preferred embodiment of the present invention, the proportion by weight of ethyl cellulose to the total weight of formulation is between 10 to 65%, preferably 15 to 50%, and more preferably between 20 to 40%.

According to another preferred embodiment of the present invention, said quetiapine is in the form of a fumarate salt thereof.

According to another preferred embodiment of the present invention, excipients used comprise at least one or a mixture of fillers, buffering agents, glidants, lubricants.

According to a preferred embodiment of the present invention, said filler is at least one of microcrystalline cellulose and lactose monohydrate, or a properly-proportioned mixture thereof.

According to a preferred embodiment of the present invention, said buffering agent is sodium citrate dihydrate. With using sodium citrate dihydrate in the formulation, the desired release profile can be obtained independent from the pH of the medium.

According to another preferred embodiment of the present invention, the proportion by weight of ethyl cellulose to sodium citrate dihydrate is between 0.2 to 60, preferably 1 to 50, and more preferably between 2 to 40.

According to a preferred embodiment of the present invention, said glidant is colloidal silicon dioxide.

According to a preferred embodiment of the present invention, magnesium stearate is used as a lubricant.

According to a preferred embodiment of the present invention, said tablet is uncoted tablet.

A further preferred embodiment according to the present invention provides a method for preparing a pharmaceutical formulation, this method comprising the steps of
a. mixing quetiapine, filler and controlled-release agent together,
b. forming wet granulation with an alcohol-water mixture,
c. wet milling and drying,
d. sieving the granules,
e. adding lubricant-glidant into the sieved powder mixture obtained with granular structure and mixing the latter,
f. compressing into tables, and
g. coating the tablets.

According to another preferred embodiment of to the present invention, said pharmaceutical formulation is consisting of
a. core
   a. quetiapine fumarate at 33 to 70% by weight
   b. ethyl cellulose at 10 to 65% by weight
   c. sodium citrate dihydrate at 2 to 30% by weight
   d. lactose monohydrate at 3.0 to 50% by weight
   e. microcrystalline cellulose at 4.0 to 60% by weight
   f. colloidal silicone dioxide at 0.2 to 5.0% by weight
   g. magnesium stearate at 0.1 to 5.0% by weight
b. coating
   a. hydroxymethylpropyl cellulose or polyvinyl alcohol at 1.0 to 5.0% by weight.

### Detailed Description of Invention

### Example

| **Content** | **Amount (%) (w/w)** |
|---|---|
| quetiapine fumarate | 51.2 |
| ethyl cellulose (ethoxyl content 48 - 52.5%) | 22.0 |
| sodium citrate dihydrate | 3.0 |
| lactose monohydrate | 4.6 |
| microcrystalline cellulose | 16.2 |
| colloidal silicone dioxide | 0.6 |
| magnesium stearate | 2.4 |

Ethyl celluloses, commercially available under the trademark Aqualon®, used in the present invention has four different types according their ethoxyl content.

| **Type** | **Ethoxyl content (%)** |
|---|---|
| K type | 45 - 47,2 |
| N type | 48 - 49, 5 |
| T type | 49,6 - 51,5 |
| X type | 50,5 - 52,5 |

(see the 2002-document specifying physical and chemical specifications of the product Aqualon ® of the company Herkules, p. 3.)
Ehtyl celluloses with type K, N, T, X, as indicated in the table, have different ethoxyl contents and show characteristic features in line with content rates. The type K, among these 4 types of ethyl cellulose gelates in organic solvents (toluene, xylene, butyl acetate, carbon tetrachloride), but the types N, T, and X does not gelate. With the invention made, at least one or a properly-proportioned mixture of the type N, T, and X ethyl celluloses are used.

The N type and/or T type ethyl cellulose used in the formulation does not dissolve in water and becomes not gelated. Specifically-selected excipients are used together with the N type and/or T type ethyl cellulose in the formulation. Sodium citrate dihydrate, among these excipients, generates a proper pH medium around the ethyl cellulose matrix and facilitates its solubility. Thanks to this feature, the release profile of the formulation is brought to the desired level independently from the pH of the medium.

Thanks to the present invention developed, a stable controlled-release formulation is surprisingly obtained thanks to making use of ethyl cellulose with an ethoxyl content of 48 - 52.5% in which quetiapine is uniformly distributed, thereby solving said problems and allowing to achieve the desired release profile. Thanks to the formulation developed, the risk of dose damping is eliminated and a controlled-release formulation is obtained by using non-gelling ethyl cellulose. Preferably core of said formulation comprise a non-gelling ethyl cellulose with an ethoxyl content of 48 - 51,5 %. More preferably core of said formulation comprise a non-gelling ethyl cellulose with an ethoxyl content of 48 - 49,5 %.

The formulation is made as follows. Quetiapine, filling agent, and controlled-release agent are mixed together. Then this mixture is subjected to a wet milling process, together with an alcohol-water mixture. Then, the granules obtained by wet milling and then dried are sieved. Thereafter, lubricant-glidant is added to the sieved powder mixture with a granular form and the resulting mixture is mixed again. Finally, the mixture obtained is compressed into tablets and the tablets obtained are coated.

Another preferred application in relation to the formulation is that they are preferred in dependence on the viscosity ranges of ethyl cellulose used. The N type ethyl cellulose selected for use in one embodiment has the following viscosity ranges.

| **Type** | **Viscosity range (mPa.s)** |
|---|---|
| N7 Pharm | 6 - 8 |
| N10 Pharm | 8 - 11 |
| N14 Pharm | 12 - 16 |
| N22 Pharm | 18 - 24 |
| N50 Pharm | 40 - 52 |
| N100 Pharm | 80 - 105 |
| T 10 Pharm | 8 - 11 |
| T 50 Pharm | 40 - 52 |

In the subject formulations are employed the N type ethyl cellulose with a 18-24 mPa.s viscosity range, among the specified N type products with a viscosity range of 5.6 to 105 mPa.s, and the T type ethyl cellulose with a 8-11 mPa.s viscosity range among the specified T type products.

It is further possible to use the following additional excipients in the formulation.

Suitable binders include, but are not restricted to at least one or a mixture of polyvinylpyrrolidone, gelatin, sugars, glucose, natural gums, gums, synthetic celluloses, polymethacrylate, hydroxypropyl methyl cellulose, hydroxypropyl cellulose, carboxymethyl cellulose, methyl cellulose, and other cellulose derivatives.

Suitable glidants include, but are not restricted to at least one or a mixture of colloidal silicone dioxide, talk, aluminum silicate.

Suitable lubricants include, but are not restricted to at least one or a mixture of sodium stearil fumarate, magnesium stearate, polyethylene glycol, stearic acid, metal stearates, boric acid, sodium chloride benzoate and acetate, sodium or magnesium lauryl sulfate.

Suitable colorants include, but are not restricted to at least one or a mixture of food, drug, and cosmetic (FD&C) dyes (FD&C blue, FD&C green, FD&C red, FD&C yellow, FD&C lake), ponceau, indigo Drug & cosmetic (D&C) blue, indigotine FD&C blue, carmoisine indigotine (indigo Carmine); iron oxides (e.g. iron oxide red, yellow, black), quinoline yellow, flame red, brilliant red (carmine), carmoisine, sunset yellow.

Treatment of diseases, such as bipolar disease, obsessive-compulsive disorder and schizophrenia, mania, depression, dementia, agitation disorders can be provided with the formulation made according to the present invention.

## Claims

1. A pharmaceutical formulation in the form of a tablet comprising a core and an optional coating on said core comprising quetiapine or a pharmaceutically acceptable salt, solvate, or polymorph thereof, **characterized in that** the core of said formulation comprises a non-gelling ethyl cellulose with an ethoxyl content of 48 to 51.5% wherein the formulation comprises ethyl cellulose of the following grades:
• ethyl cellulose with an ethoxyl content of 48 to 49.5% having a viscosity of 18 to 24 mPa.s, or
• ethyl cellulose with an ethoxyl content of 49.6 to 51.5% having a viscosity of 8 to 11 mPa.s,
wherein the weight ratio of quetiapine to ethyl cellulose is between 1.5 to 3, and wherein the proportion by weight of ethyl cellulose to the total weight of formulation is between 20 to 40%.

2. The pharmaceutical formulation according to Claim 1, wherein the core of said formulation comprises a non-gelling ethyl cellulose with an ethoxyl content of 48 to 49.5%.

3. The pharmaceutical formulation according to any of the preceding claims, wherein said quetiapine is in the form of its fumarate salt.

4. The pharmaceutical formulation according to any of the preceding claims, further containing excipients, comprising at least one or a mixture of fillers, buffering agents, glidants, lubricants.

5. The pharmaceutical formulation according to any of the preceding claims, wherein said filler is at least one of microcrystalline cellulose and lactose monohydrate, or a properly-proportioned mixture thereof.

6. The pharmaceutical formulation according to any of the preceding claims, wherein said buffering agent is sodium citrate dihydrate.

7. The pharmaceutical formulation according to any of the preceding claims, wherein the weight ratio of ethyl cellulose to sodium citrate dihydrate is between 0.2 to 60, preferably 1 to 50, and more preferably between 2 to 40.

8. The pharmaceutical formulation according to any of the preceding claims, wherein said glidant is colloidal silicone dioxide.

9. The pharmaceutical formulation according to any of the preceding claims, wherein said lubricant is magnesium stearate.

10. A method for preparing a pharmaceutical formulation according to any of the preceding claims, comprising the steps of:
a. mixing quetiapine, filler and controlled-release agent together, wherein said controlled-release agent is a non-gelling ethyl cellulose with an ethoxyl content of 48 to 51.5%, whereby ethyl cellulose is of the type having an ethoxyl content of 48-49.5% with a viscosity of 18 to 24 mPa.s, or ethyl cellulose having an ethoxyl content of 49.6 to 51.5% with a viscosity of 8 to 11 mPa.s,
b. forming wet granulation with an alcohol-water mixture,
c. wet milling and drying,
d. sieving the granules,
e. adding lubricant-glidant into the sieved powder mixture obtained with granular structure and mixing the latter,
f. compressing into tablets, and
g. coating the tablets.

11. The pharmaceutical formulation according to any of the preceding claims, consisting of:
a. core
a. quetiapine fumarate at 33 to 70% by weight
b. ethyl cellulose at 20 to 40% by weight
c. sodium citrate dihydrate at 2 to 30% by weight
d. lactose monohydrate at 3.0 to 50% by weight
e. microcrystalline cellulose at 4.0 to 60% by weight
f. colloidal silicone dioxide at 0.2 to 5.0% by weight
g. magnesium stearate at 0.1 to 5.0% by weight
b. coating
a. hydroxymethylpropyl cellulose or polyvinyl alcohol at 1.0 to 5.0% by weight.

12. A pharmaceutical formulation according to any of the preceding claims for use in preventing or treating bipolar disease, obsessive-compulsive disorder and schizophrenia, mania, depression, dementia, agitation disorders in mammalians and particularly in humans.

## Patentansprüche

1. Pharmazeutische Formulierung in Form einer Tablette, die einen Kern und eine optionale Beschichtung auf dem Kern umfasst, umfassend Quetiapin oder ein pharmazeutisch verträgliches Salz, Solvat oder Polymorph davon, **dadurch gekennzeichnet, dass** der Kern der pharmazeutischen Formulierung eine nicht-gelierende Ethylcellulose mit einem Ethoxylgehalt von 48 bis 51,5 % umfasst, wobei die Formulierung Ethylcellulose mit den folgenden Klassifikationen umfasst:
• Ethylcellulose mit einem Ethoxylgehalt von 48 bis 49,5 % mit einer Viskosität von 18 bis 24 mPas, oder
• Ethylcellulose mit einem Ethoxylgehalt von 49,6 bis 51,5 % mit einer Viskosität von 8 bis 11 mPas,
wobei das Gewichtsverhältnis von Quetiapin zur Ethylcellulose zwischen 1,5 bis 3 beträgt, und
wobei der Gewichtsanteil von Ethylcellulose zwischen 20 bis 40 % des Gesamtgewichts der Formulierung beträgt.

2. Pharmazeutische Formulierung nach Anspruch 1, wobei der Kern der Formulierung eine nicht-gelierende Ethylcellulose mit einem Ethoxylgehalt von 48 bis 49,5 % umfasst.

3. Pharmazeutische Formulierung nach einem der vorangehenden Ansprüche, wobei das Quetiapin in Form seines Fumaratsalzes vorliegt.

4. Pharmazeutische Formulierung nach einem der vorangehenden Ansprüche, die des Weiteren Exzipienten enthält, die mindestens eines von oder ein Gemisch an Füllmitteln, Pufferagenzien, Gleitmitteln, Schmiermitteln umfassen.

5. Pharmazeutische Formulierung nach einem der vorangehenden Ansprüche, wobei das Füllmittel mindestens eines von mikrokrystalliner Cellulose und Laktosemonohydrat oder ein angemessenes proportioniertes Gemisch davon ist.

6. Pharmazeutische Formulierung nach einem der vorangehenden Ansprüche, wobei das Pufferagens Natriumcitratdihydrat ist.

7. Pharmazeutische Formulierung nach einem der vorangehenden Ansprüche, wobei das Gewichtsverhältnis von Ethylcellulose zu Natriumcitratdihydrat zwischen 0,2 bis 60, vorzugsweise 1 bis 50 und mehr bevorzugt zwischen 2 bis 40 beträgt.

8. Pharmazeutische Formulierung nach einem der vorangehenden Ansprüche, wobei das Gleitmittel kolloidales Siliziumdioxid ist.

9. Pharmazeutische Formulierung nach einem der vorangehenden Ansprüche, wobei das Schmiermittel Magnesiumstearat ist.

10. Verfahren zur Herstellung einer pharmazeutischen Formulierung nach einem der vorangehenden Ansprüche, umfassend die Schritte:
a. Mischen von Quetiapin, Füllstoff und Agens zur kontrollierten Freisetzung, wobei das Agens zur kontrollierten Freisetzung eine nicht-gelierende Ethylcellulose mit einem Ethoxylgehalt von 48 bis 51,5 % ist, wobei die Ethylcellulose derart ist, dass sie einen Ethoxylgehalt von 48 bis 49,5 % mit einer Viskosität von 18 bis 24 mPas hat oder Ethylcellulose mit einem Ethoxylgehalt von 49,6 bis 51,5 % mit einer Viskosität von 8 bis 11 mPas hat,
b. Nassgranulation mit einem Alkohol-Wasser-Gemisch,
c. Nassvermahlung und Trocknen,
d. Sieben der Granula,
e. Hinzufügen von Schmiermittel-Gleitmittel in das gesiebte Pulvergemisch, das mit körniger Struktur erhalten wurde, und Mischen des Letzteren,
f. Komprimieren zu Tabletten, und
g. Beschichten der Tabletten.

11. Pharmazeutische Formulierung nach einem der vorangehenden Ansprüche, die besteht aus:
a. Kern
a. Quetiapinfumarat mit 33 bis 70 Gew.-%
b. Ethylcellulose mit 20 bis 40 Gew.-%
c. Natriumcitratdihydrat mit 2 bis 30 Gew-%.
d. Laktosemonohydrat mit 3,0 bis 50 Gew.-%
e. mikrokristalliner Cellulose mit 4,0 bis 60 Gew.-%
f. kolloidalem Siliziumdioxid mit 0,2 bis 5,0 Gew.-%
g. Magnesiumstearat mit 0,1 bis 5,0 Gew.-%
b. Beschichtung
a. Hydroxymethylpropylcellulose oder Polyvinylalkohol mit 1,0 bis 5,0 Gew.-%.

12. Pharmazeutische Formulierung nach einem der vorangehenden Ansprüche zur Verwendung bei der Prophylaxe oder der Behandlung von bipolarer Erkrankung, Zwangserkrankung und Schizophrenie, Manie, Depression, Demenz, Agitation-Störungen bei Säugern und insbesondere bei Menschen.

## Revendications

1. Formulation pharmaceutique sous la forme d'un comprimé comprenant un coeur et un enrobage facultatif sur ledit coeur comprenant de la quétiapine ou un sel pharmaceutiquement acceptable, un solvate, ou un polymorphe de celle-ci, **caractérisée en ce que** le coeur de ladite formulation comprend une éthylcellulose non gélifiante ayant une teneur en éthoxyle de 48 à 51,5 % dans laquelle la formulation comprend une éthylcellulose répondant aux qualités suivantes :
• éthylcellulose ayant une teneur en éthoxyle de 48 à 49,5 % avec une viscosité de 18 à 24 mPa.s, ou
• éthylcellulose ayant une teneur en éthoxyle de 49,6 à 51,5 % avec une viscosité de 8 à 11 mPa.s,
dans laquelle le rapport en poids de la quétiapine à l'éthylcellulose est compris entre 1,5 et 3, et
dans laquelle la proportion en poids d'éthylcellulose par rapport au poids total de la formulation est comprise entre 20 et 40 %.

2. Formulation pharmaceutique selon la revendication 1, dans laquelle le coeur de ladite formulation comprend une éthylcellulose non gélifiante ayant une teneur en éthoxyle de 48 à 49,5 %.

3. Formulation pharmaceutique selon l'une quelconque des revendications précédentes, dans laquelle ladite quétiapine est sous la forme de son sel de fumarate.

4. Formulation pharmaceutique selon l'une quelconque des revendications précédentes, contenant en outre des excipients, dont au moins une charge ou un mélange de charges, des agents de tamponnage, des agents glissants, des lubrifiants.

5. Formulation pharmaceutique selon l'une quelconque des revendications précédentes, dans laquelle ladite charge est au moins une charge parmi une cellulose microcristalline et un monohydrate de lactose, ou par un mélange correctement proportionné de ceux-ci.

6. Formulation pharmaceutique selon l'une quelconque des revendications précédentes, dans laquelle ledit agent de tamponnage est un dihydrate de citrate de sodium.

7. Formulation pharmaceutique selon l'une quelconque des revendications précédentes, dans laquelle le rapport en poids de l'éthylcellulose au dihydrate de citrate de sodium est compris entre 0,2 et 60, de préférence entre 1 et 50, et plus préférablement entre 2 et 40.

8. Formulation pharmaceutique selon l'une quelconque des revendications précédentes, dans laquelle ledit agent glissant est un dioxyde de silicone colloïdale.

9. Formulation pharmaceutique selon l'une quelconque des revendications précédentes, dans laquelle ledit lubrifiant est le stéarate de magnésium.

10. Méthode de préparation d'une formulation pharmaceutique selon l'une quelconque des revendications précédentes, comprenant les étapes suivantes :
a. mélange de la quétiapine, de la charge et d'un agent de libération contrôlée, dans lequel ledit agent de libération contrôlée est une éthylcellulose non gélifiante ayant une teneur en éthoxyle de 48 à 51,5 %, ladite éthylcellulose étant du type à avoir une teneur en éthoxyle de 48 à 49,5 % avec une viscosité de 18 à 24 mPa.s, ou une éthylcellulose ayant une teneur en éthoxyle de 49,6 à 51,5 % avec une viscosité de 8 à 11 mPa.s,
b. formation d'une granulation par voie humide à l'aide d'un mélange alcool-eau,
c. broyage à l'état humide et séchage,
d. tamisage des granulés,
e. ajout du lubrifiant-agent glissant au mélange pulvérulent tamisé obtenu ayant une structure granulaire et homogénéisation de ce dernier,
f. compression pour obtenir des comprimés, et
g. enrobage des comprimés.

11. Formulation pharmaceutique selon l'une quelconque des revendications précédentes, constitué de :
a. coeur
a. fumarate de quétiapine à raison de 33-70 % en poids
b. éthylcellulose à raison de 20-40 % en poids
c. dihydrate de citrate de sodium à raison de 2-30 % en poids
d. monohydrate de lactose à raison de 3,0-50 % en poids
e. cellulose microcristalline à raison de 4,0-60 % en poids
f. dioxyde de silicone colloïdale à raison de 0,2-5,0 % en poids
g. stéarate de magnésium à raison de 0,1-5,0 % en poids
b. enrobage
a. hydroxyméthylpropylcellulose ou alcool polyvinylique à raison de 1,0-5,0 % en poids.

12. Formulation pharmaceutique selon l'une quelconque des revendications précédentes destinée à être utilisée pour prévenir ou traiter les troubles bipolaires, les troubles obsessionnels compulsifs et la schizophrénie, la manie, la dépression, la démence, un trouble du comportement de type agitation chez les mammifères et en particulier chez l'homme.
